# FASCICULE DE BREVET EUROPEEN

(11) **EP 2 310 348 B1**
(45) Date de publication et mention de la délivrance du brevet: **15.04.2015**
(21) Numéro de dépôt: 09737097.7
(22) Date de dépôt: 21.07.2009
(51) Int. Cl.: C07C 41/50, C07C 43/30, C07C 43/303

(54) **PROCEDE DE FABRICATION DE DIALCOXYALCANES PAR OXYDATION PARTIELLE D'ALCOOLS INFERIEURS EN PRESENCE D'UN CATALYSEUR A BASE DE MOLYBDENE ET FER**
VERFAHREN ZUR HERSTELLUNG VON DIALKOXYALKANEN DURCH PARTIELLE OXIDATION NIEDERER ALKOHOLE IN GEGENWART EINES AUF MOLYBDEN UND EISEN BASIERENDEN KATALYSATORS
METHOD FOR PRODUCING DIALKOXY ALKANES BY PARTIAL OXIDATION OF LOWER ALCOHOLS IN THE PRESENCE OF A CATALYST BASED ON MOLYBDENUM AND IRON

(30) Priorité: 22.07.2008 FR 0854966
(43) Date de publication de la demande: 20.04.2011
(73) Titulaire: Arkema France, 92700 Colombes (FR)
(72) Inventeur: DUBOIS, Jean-Luc, F-69390 Millery (FR)
(74) Mandataire: Killis, Andréas
(86) Numéro de dépôt international: PCT/FR2009/051456
(87) Numéro de publication internationale: WO 2010/010287

(56) Documents cités:
- WO-A-99/52630
- WO-A-2007/034264
- WO-A-2007/128941

## Description

La présente invention porte sur un procédé de fabrication de dialcoxyalcanes par oxydation partielle directe d'un alcool léger avec un catalyseur à base d'oxyde mixte comprenant du Molybdène et du fer.

Les dialcoxyalcanes du procédé de l'invention répondent à la formule générale suivante : RR'CH-O-CRR'-O-CHRR' dans laquelle R et R' sont soit H, soit un radical CH₃-(CH₂)ₙ-, n étant compris entre 0 et 2 et tel que le nombre total d'atomes de carbone des radicaux R et R' est < à 3.

Ces composés sont obtenus par oxydation des alcools légers c'est-à-dire les alcools linéaires comportant de 1 à 4 atomes de carbone. Il s'agit des alcools primaires tels que le méthanol, l'éthariol, le 1-propanol, le 1-butanol ou des alcools secondaires tels que le, 2-propanol (ou isopropanol) ou le 2-butanol.

Lorsque la réaction de synthèse est mise en oeuvre avec des alcools primaires la formule générale des dialcoxyalcanes est simplifiée : RCH₂-O-CHR-O-CH₂R. C'est celle des dialcoxyalcanes les plus recherchés industriellement à savoir le diméthoxyméthane (ou méthylal) et le 1,1-diéthoxyéthane (ou acétal).

Les procédés d'oxydation des alcools et notamment des mono-alcools légers sont bien connus depuis un siècle au moins.

Cette oxydation peut emprunter deux voies se distinguant par le mécanisme réactionnel mis en oeuvre. La première voie est l'oxydation «simple» qui sera l'objet des développements ci-dessous et la seconde voie celle de la déshydrogénation.

Cette seconde voie peut être conduite sous forme de déshydrogénation non oxydante selon le mécanisme réactionnel suivant : RCH₂OH → RCHO + H₂ (en défaut d'oxygène) avec donc production d'hydrogène, ou sous forme de la déshydrogénation oxydante (oxydéshydrogénation) et avec production d'eau par oxydation de l'hydrogène. Ces réactions sont conduites en phase gazeuse en présence par exemple d'un catalyseur au cuivre réduit ou à l'argent métal à des températures généralement comprises entre 600 et 700 °C. On peut se référer à ce sujet à des ouvrages comme celui de l'Institut Français du Pétrole « catalyse de Contact » paru aux Editions Technip (1978) pages 385-393 ou le Catalyst Handbook M.V. Twigg publié par Wolfe Publishing Ltd (1989) pages 490 à 503. Ces procédés sont généralement utilisés pour synthétiser des aldéhydes (formol à partir de méthanol) ou des acides ou des esters.

En ce qui concerne la première voie d'oxydation simple à l'oxygène, il est bien connu que l'oxydation du méthanol en présence de catalyseurs conduit à basse température à l'obtention d'un mélange de divers composés oxydés tels que notamment le formaldéhyde, le formiate de méthyle ou le méthylal (diméthoxyméthane).

On peut illustrer les diverses réactions catalytiques alors mises en jeu avec le méthanol par le schéma suivant :

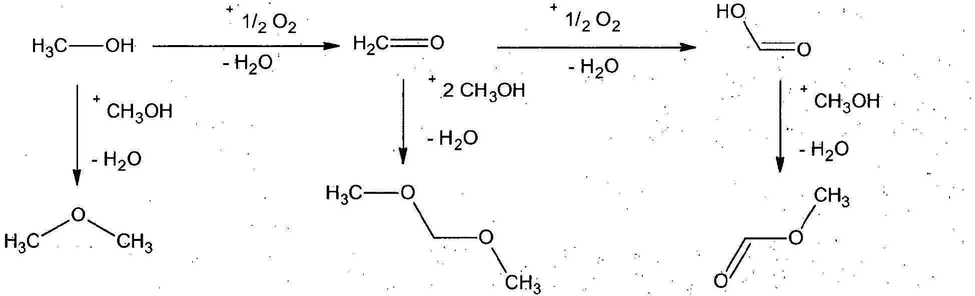

Le même schéma peut être transposé à l'éthanol et aux autres alcools légers.

Les procédés classiques visant la production de l'aldéhyde par une oxydation partielle de l'alcool répondent donc à la réaction suivante dans le cas des alcools primaires :

2 RCH₂OH + O₂ → 2 RCHO + 2H₂O

Les procédés d'oxydation profonde des alcools légers permettent de synthétiser des acides (puis les esters correspondants) selon la réaction globale suivante :

2 RCH₂OH + 2 O₂ → 2 RCOOH + 2 H₂O

qui est le résultat des deux étapes suivantes :

2 RCH₂OH + O₂ → 2 RCHO + 2H₂O

2 RCHO + O₂ → 2 RCOOH,

suivie de l'estérification

2 RCOOH + 2 RCH₂OH → 2 RCOO CH₂R + 2 H₂O.

Au contraire des procédés de la seconde voie, les procédés d'oxydation partielle des alcools légers permettent également de former des dialcoxyalcanes selon la réaction globale suivante correspondant aux alcools primaires :

6 RCH₂OH + O₂ → 2 RCH₂ORCHOCH₂R + 4H₂O

qui est le résultat de deux étapes successives :

2 RCH₂OH + O₂ → 2 RCHO + 2H₂O

2 RCHO + 4 RCH₂0H → 2 RCH₂ORCHOCH₂R+2H₂O

Il est usuel de distinguer d'une part les oxydation totales ou profondes qui permettent de former les acides ou esters et les oxydations partielles qui s'arrêtent au stade de l'aldéhyde ou dialcoxyalcane.

La concomitance de ces différentes réactions et la présence dans le milieu des diverses molécules est illustrée par exemple par les articles de N. Pernicone et al dans «On the Mechanism of CH3OH Oxidation to CH2O over MoO3-Fe2 (MoO4)3 Catalyst » paru dans Journal of Catalysis 14, 293-302 (1969) et de Haichao Liu and Enrique Iglesia publié dans J. Phys. Chenu. B (2005), 109, 2155-2163 « Selective Oxidation of Methanol and Ethanol on Supported Ruthenium Oxide Clusters at Low Temperatures ».

Des mécanismes analogues sont mis en oeuvre dans les réactions d'oxydation des alcools légers secondaires tels que le 2-propanol et le 2-butanol.

L'oxydation initiale de l'alcool conduit à une cétone de formule CH₃-CO-CH₃ avec l'isopropanol et CH₃-CO-C₂H₅ avec le 2-butanol. L'étape suivante de réaction de la cétone avec l'alcool léger conduit aux dialcoxyalcanes de formules respectives (CH₃)₂CH-OC(CH₃)₂-O-CH(CH₃)₂ et (C₂H₅)(CH₃)CH-O-C(CH₃)(C₂H₅)-O-CH(CH₃)(C₂H₅). La réaction globale pour l'oxydation en dialcoxyalcane 2,2-diisopropoxypropane de l'isopropanol se résume comme suit.

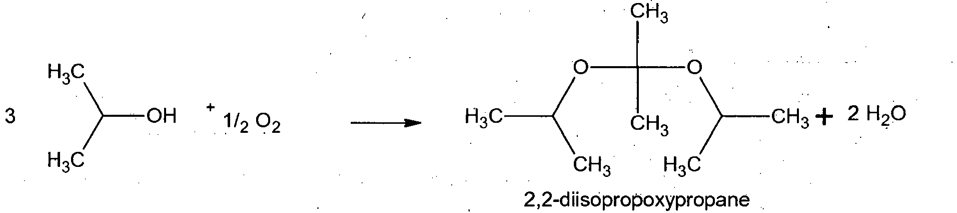

Les travaux de recherche ayant un objectif industriel se sont donc tournés vers l'étude des conditions opérationnelles, température, phase liquide ou phase gazeuse et surtout des catalyseurs du procédé permettant d'obtenir le composé oxydé « cible », aldéhyde, acide et/ou ester ou dialcoxyalcane. Le problème à résoudre est de parvenir par oxydation directe de la charge d'alcool, au produit « cible » recherché avec, à la fois, une conversion et une sélectivité élevées.

Les procédés industriels classiques de production de l'aldéhyde par l'oxydation classique (première voie) répondent à la réaction suivante :

2 RCH₂OH + O₂ → 2 RCHO + 2H₂O

Cette oxydation est réalisée en phase gazeuse en présence de catalyseurs de type oxydes mixtes à une température comprise entre 200 et 400 °C. Dans ce dernier cas, l'oxygène présent au sein du milieu réactionnel est en excès mais utilisé sous forme diluée, les pressions partielles en O₂ et alcool sensiblement égales sont de l'ordre de quelques % présentant donc un ratio molaire O₂/Alcool > 1, l'essentiel du milieu réactionnel étant constitué d'inerte afin de ne pas être dans des conditions inflammables. L'utilisation d'un excès stoechiométrique important d'oxygène à température relativement élevée peut conduire, si l'on ne prend pas de précautions, à une oxydation profonde et donc à l'acide homologue de l'alcool (voir schéma précédent) par oxydation de l'aldéhyde, la réaction pouvant d'ailleurs aller encore plus loin pour conduire à la « combustion » de l'acide en produisant du gaz carbonique et de l'eau.

La fabrication du formol ou formaldéhyde était, et est toujours, un secteur particulièrement attractif ce qui explique l'abondance de la littérature sur ce sujet alors que les procédés de base remontent au début du siècle dernier pour la voie déshydratation et à 1931 pour la voie oxydation.

L'article de N. Pernicone et al mentionné ci-dessus fait référence à un procédé de synthèse industriel du formaldéhyde, procédé Montedison, catalysé par un oxyde mixte à base de Molybdène et de Fer et fait état d'une étude sur le mécanisme réactionnel de ce type de réactions y compris les réactions parasites secondaires.

On peut citer également le brevet US 7,468,341 qui décrit un catalyseur d'oxydation du méthanol en formaldéhyde constitué par un oxyde mixte Fe-Mo associé à un oxyde mixte contenant du Cérium ou encore la demande WO 99/52630 qui vise dans un procédé d'oxydation du méthanol en formaldéhyde de la régénération in situ du catalyseur molybdate de fer. Tout ce qui précède illustre le rôle essentiel que ce type de catalyseurs jouent sur le plan industriel dans la fabrication du formaldéhyde.

Les travaux conduits pour la synthèse de composés spécifiques (cibles) d'oxydation des alcools ont essentiellement porté sur l'étude des types de catalyseurs adaptés à la mise en oeuvre de telle oxydation spécifique. On peut relever à côté de la synthèse classique d'aldéhydes les travaux suivants.

Pour l'oxydation profonde conduisant à l'acide formique ou son ester, le formiate de méthyle, on peut citer la demande de brevet US 2005/0059839 A1 qui décrit des catalyseurs d'oxydation du méthanol constitués par des métaux du groupe du platine (le ruthénium) déposés sur support. Cette demande de brevet correspond aux travaux de H. Liu and E. Iglesia visés dans la publication citée ci-dessus.

Des travaux spécifiques ont été conduits sur les procédés d'oxydation partielle des alcools pour sur la synthèse de méthylal et portaient notamment sur les catalyseurs à mettre en oeuvre dans ce type de procédé.

On peut citer les documents suivants.

Le brevet US-A-2 663 742 décrit un procédé de fabrication du méthylal par oxydation en phase vapeur du méthanol en présence d'un catalyseur et d'un halogène ou d'une halogénure d'hydrogène.

Plusieurs recherches se sont focalisées sur l'utilisation de catalyseurs à base de Rhénium. Le brevet US 6,403,841 décrit un procédé de fabrication du méthylal par oxydation du méthanol sur un catalyseur à base rhénium-antimoine (SbRe₂O₆). La réaction est réalisée avec un excès d'oxygène en présence d'un important volume de gaz inerte (en volume : 5 % méthanol, 10 % oxygène et 85 % hélium, ratio O₂/Méthanol = 2). Ces travaux réalisés par Y.Yuan, et al. ont fait l'objet de plusieurs publications telles que Chem. Comm., 2000, 1421-1422, qui décrit des catalyseurs à base de rhénium supporté ou non ainsi que dans: J. Phys. Chem. B, 2002, 106, 4441; Topics in Catalysis, Vol 22, No 1/2, January 2003 ; Chemistry Letters 2000, 674 *and* J. Catal. 195 (2000) 51-61.

D'autres travaux ont été conduits sur l'utilisation de catalyseurs à base de molybdène.

La demande US n° 2005/0154226 A1. décrit un procédé de production de méthylal par oxydation de méthanol et/ou du diméthylether. La réaction est réalisée sur un catalyseur d'un hétéropolyacide de formule H₃₊ₙXVₙMo₁₂₋ₙO₄₀, où X représente le phosphore ou le silicium, et n une valeur de 0 à 4. Les meilleurs résultats semblent être obtenus avec un catalyseur H₅PV₂Mo₁₀O₄₀ sur silice. Ces travaux ont aussi été publiées, dans J. Phys. Chem. B 2003, 107, 10840-10847.

M. Fournier, C. Rocchicciolo-Deltcheff, et al. décrivent l'évaluation de catalyseurs de formule H₃PMo₁₂O₄₀ / silice, pour l'oxydation du méthanol en méthylal (J. Chem. Soc., Chem. Commun. 1994, 307-308). La même équipe décrit l'utilisation dans la même réaction d'un catalyseur de formule H₄SiMo₁₂O₄₀ / silice (J. Chem. Soc., Chem. Commun. 1998, 1260-1261).

La demanderesse a déposé une demande de brevet WO2007/034264 décrivant l'utilisation dans ce type de procédé d'oxydation partielle d'un alcool léger d'un catalyseur constitué d'un oxyde mixte à base de molybdène et de vanadium associés le cas échéant à d'autres éléments métalliques. Le catalyseur préféré répondait à la formule Mo₁₂ V3 W_{1.2} Cu_{1.2} Sb_{0.5} Oₓ, x étant une valeur numérique déterminée par le degré d'oxydation des autres éléments. Ce type de catalyseur permet notamment d'obtenir des rendements élevés en acétals dans une large gamme de pressions partielles en méthanol et dans une large gamme de ratio O₂/Méthanol.

Par ailleurs, la demanderesse a également déposé une demande de brevet WO2007/128941 qui décrit un procédé catalytique d'oxydation partielle d'un, alcool léger mettant en oeuvre un alcane léger comme gaz inerte de dilution du milieu réactionnel. Ce type de procédé peut être utilisé pour la synthèse de méthylal avec le catalyseur de la demande de brevet WO2007/034264.

Par ailleurs, J. Sambeth, L. Gambaro and H. Thomas, Adsorption Science Technology (1995) page 171, utilisent le péntoxyde de vanadium pour l'oxydation du méthanol, le méthylal étant l'un des produits issus de la réaction.

Aucun des catalyseurs connus pour la préparation d'un produit d'oxydation partielle d'un alcool léger sous forme d'un dialcoxyalcane tel que par exemple le méthylal par oxydation directe du méthanol, ne donne une complète satisfaction.

La présente invention a pour objet de pallier à ces inconvénients et propose un procédé de synthèse d'un dialcoxyalcane par oxydation partielle, directe d'un alcool léger permettant d'atteindre à la fois des rendements, des productivités et des sélectivités élevées en dialcoxyalcane.

La présente invention a donc pour objet un procédé de fabrication d'un produit d'oxydation partielle d'un alcool léger, sous forme d'un dialcoxyalcane dans lequel un alcool léger comprenant de 1 à 4 atomes de carbone est soumis à une oxydation par contact en phase gazeuse avec de l'oxygène ou un gaz contenant de l'oxygène moléculaire en présence d'un catalyseur répondant à la composition suivante :

**Mo₁₂ Feₐ X¹_{b} X²_{c} X³_{d} X⁴ₑ X⁵_{f} Oₓ**

dans laquelle Mo = molybdène ; O = oxygène ; Fe = fer ; X¹ = au moins un élément choisi parmi le chrome, le nickel, le cobalt, le manganèse, l'étain et le cuivre, X² = au moins un élément choisi parmi le bismuth, l'antimoine, le tellure, l'indium, l'aluminium et le silicium , X³ = au moins un élément choisi parmi le phosphore, le tungstène, le titane, le vanadium, le tantale et le niobium; X⁴ = au moins un élément choisi parmi les métaux alcalino-terreux, le lanthane ou le cérium; X⁵ est au moins un élément choisi parmi les métaux alcalins ; et a, b, c d, e sont des indices dont les valeurs sont 1,5≤a≤8 ; 0≤b≤4 ; 0≤c≤5 ; 0≤d≤2 ; 0≤e≤2 ; 0<f<2 et x est une valeur numérique déterminée par le degré d'oxydation des autres éléments, et caractérisé par le fait que, au sein du milieu réactionnel la pression partielle en alcool est comprise entre 15 et 80 % et de préférence comprise entre 20 et 50 % et celle de l'oxygène comprise entre 2 et 20 %, le ratio des pressions. partielles O₂/Alcool étant inférieur ou égal à 1 et de préférence compris entre 0,5/6 et 1, le reste du milieu étant constitué d'un gaz inerte vis à vis de la réaction.

Un alcool léger dans le procédé de la présente invention désigne un alcool linéaire ayant 1 à 4 atomes de carbone, autrement dit le méthanol, l'éthanol, le propanol et le butanol, la fonction alcool étant placée en 1 ou en 2 pour ces deux derniers.

Dans le catalyseur le rapport atomique Mo/Fe sera compris entre 1,5 et 8 et de préférence entre 2,5 et 4,5 pour assurer au catalyseur industriel pour une meilleure durée de vie et une meilleure stabilité. Dans le procédé de l'invention on utilisera des oxydes mixtes de molybdène et de fer qui pourront être associés à au moins un métal susceptible de prendre le degré d'oxydation trois tels que le bismuth, l'aluminium, le chrome, l'indium, l'antimoine et le tellure, et/ou au moins un métal choisi parmi le phosphore, le tungstène, le vanadium, le nickel, le cobalt, le cuivre, le titane, le tantale, le niobium, le manganèse, l'étain et le silicium qui joue en général plus le rôle d'un liant que d'un composant de la phase active du catalyseur.

Les catalyseurs préférés du procédé de l'invention seront ceux qui associeront sous forme d'oxydes mixtes, le molybdène et le fer ou le molybdène, le fer et le bismuth. On peut citer par exemple les oxydes mixtes de formules : MoO₃₋Fe₂(MoO₄)₃, Mo₁₂BiFe_{3.7}Co_{4.7}Ni_{2.6}K_{0.09}Sb₁Si_{7.9}Oₓ ou Mo₁₂BiFe_{3.7}Co_{4.7}Ni_{2.6}K_{0.09}Ti_{0.5}Si₁₉Oₓ.

Pour réaliser l'oxydation de l'alcool léger on introduit dans le réacteur contenant le catalyseur une charge gazeuse de départ composée d'un mélange de l'alcool léger gazeux à oxyder, de l'oxygène moléculaire ou un gaz contenant de l'oxygène moléculaire, tel que l'air, ainsi qu'éventuellement un gaz diluant (autre que l'azote de l'air). Pour parvenir aux domaines de compositions définies ci-dessus on utilisera de préférence de l'air dilué ou un mélange alcool-air tout en veillant à la présence d'un excès d'oxygène par rapport à la stoechiométrie de la réaction pour éviter la dégradation du catalyseur.

La charge gazeuse sera constituée d'un mélange d'alcool léger et d'oxygène généralement en présence d'un gaz inerte, l'azote de l'air le plus souvent, ayant une teneur élevée en alcool telle que la pression partielle en alcool au sein du milieu réactionnel est supérieure à 15 et inférieure ou égale à 80 % et de préférence comprise entre 20 et 50 % et celle de l'oxygène comprise entre 2 et 20 %.

La concentration de l'alcool léger dans le courant gazeux, exprimée en pression partielle, est avantageusement comprise entre 25 et 40 %, de préférence entre 30 à 37 %. On utilisera de préférence, un mélange d'air et de l'alcool à oxyder pour simplifier et optimiser les conditions opérationnelles en évitant autant que possible le recyclage des coproduits CO, CO₂, N₂ de la réaction.

Le rapport molaire de l'oxygène (calculé en tant qu'O₂) à l'alcool léger est inférieur à 1 et de préférence compris entre 0,5/6 et 1 /1. Le choix des quantités respectives d'oxygène et d'alcool dépend du type de mise en oeuvre du procédé soit en recherchant une conversion totale, auquel cas on doit être au-dessus de la stoechiométrie de la réaction, soit une conversion partielle pour laquelle un déficit d'oxygène par rapport à la stoechiométrie suffit. On utilisera de préférence un ratio dé 1,2/6 à 0,9/1. Le gaz contenant de l'oxygène moléculaire peut être de l'air ou de l'air enrichi en oxygène. De préférence on utilise de l'air en mélange avec l'alcool à oxyder.

La réaction réalisée en phase gazeuse sera généralement conduite à une température comprise entre 10 et 400°C et sous une pression comprise entre 50 et 1 000 kPa et avec une vitesse spatiale d'introduction du mélange réactionnel notamment comprise entre 2000 et 100 000 h⁻¹.

On conduit l'oxydation par contact en phase vapeur à une température comprise notamment entre 10 et 400 °C, de préférence entre 100 et 350 °C; et de façon davantage préférée, entre 200 et 300 °C.

On conduit l'oxydation par contact en phase vapeur à une pression généralement comprise entre 50 et 1 000 kPa, de préférence entre 100 et 500 kPa.

La vitesse spatiale d'introduction du mélange réactionnel est généralement comprise entre 2 000 et 100 000 h⁻¹, de préférence entre 11 000 et 44 000 h⁻¹.

Les dialcoxyalcanes préférés pouvant être obtenus selon le procédé de l'invention sont le diméthoxyméthane, également appelé méthylal ou formaldéhyde diméthyl acétal, et le 1,1-diéthoxyéthane ou acétal.

La présente invention porte plus particulièrement sur la préparation de ces deux alcoxyalcanes et notamment du méthylal par oxydation partielle directe (en une étape) à partir du méthanol (ou de l'éthanol) et d'oxygène ou d'un gaz contenant de l'oxygène, la stoechiométrie de la réaction globale étant la suivante :

6 CH₃OH + O₂ → 2 CH₃OCH₂OCH₃ + 4 H₂O

Cette réaction appliquée à l'oxydation de l'éthanol pour obtenir l'acétal ou 1,1-diéthoxyéthane correspond à

6 CH₃CH₂OH + O₂ → 2 CH₃CH₂OCH(CH₃)OCH₂CH₃ + 4 H₂O

En effet la déposante a découvert de manière surprenante que les catalyseurs à base d'oxyde mixte de Molybdène et de Fer, largement utilisés pour la synthèse du formaldéhyde à partir du méthanol, permettent d'obtenir par oxydation directe du méthanol des rendements élevés (conversion et sélectivité) en méthylal et qu'ils permettent aussi de synthétiser le 1,1-diéthoxyéthane à partir de l'éthanol.

En effet, la déposante a observé de manière surprenante s'agissant de catalyseurs dédiés à la synthèse de l'aldéhyde, que des rendements élevés en acétals pouvaient être obtenus en utilisant, en présence de catalyseurs tels que définis dans la formule générale définie ci-dessus, un mélange Air/Alcool à teneur élevée en alcool, supérieure à 15 %, contenant de préférence de 30 à 40 % d'alcool. Par exemple on utilisera un mélange àir/alcool à 35 % d'alcool, soit un mélange ternaire O₂/N₂/Alcool de composition proche de 13/52/35 et donc un ratio O₂/Alcool de 13/35.

Par rapport à l'art antérieur, les avantages, outre les performances en matière de rendement et de sélectivité, sont une productivité plus importante et une moindre consommation d'énergie car il n'est pas nécessaire d'utiliser un fort débit ou une forte concentration de gaz inerte diluant pour maintenir le mélange rédactionnel en dehors de la zone d'inflammabilité du mélange Alcool - Oxygène - Gaz inerte. Dans le cas de l'utilisation de gaz inerte(s) en quantité(s) suffisante(s) pour rester en dehors des limites d'inflammabilité on choisira avantageusement ceux-ci parmi l'azote, CO₂, H₂O ou CH₄. On opèrera en l'absence d'halogène ou d'halogénure d'hydrogène afin d'éviter la formation d'halogénométhane.

On peut noter que ces conditions sont fort différentes de celles décrites dans US-A-2 663 742, où le rapport molaire MeOH/O₂ est supérieur à 12 mais où on travaille en présence de chlore. Les conditions de la présente réaction sont notamment un rapport molaire MeOH/O₂ inférieur à 12 et de préférence inférieur à 6, car il est souhaité d'une part de se trouver en dehors de la zone d'inflammabilité et; d'autre part de disposer de suffisamment d'O₂ à la sortie du réacteur pour maintenir la stabilité du catalyseur, lorsque l'on travaille à conversion élevée.

Le procédé peut être mis en oeuvre dans toute technologie de réacteur utilisant un catalyseur solide qui permet d'éliminer efficacement la chaleur de réaction. On peut citer par exemple les lits fixes multitubulaires, les lits fluidisés circulants, ou encore les lits fluidisés. Le catalyseur est alors mis en forme selon la technologie de réacteur sélectionnée, par les techniques bien connues de l'homme de l'art ; par exemple sous la. forme de pastilles, d'anneaux (cylindres creux), d'extrudés massiques ou encore de catalyseurs supportés sur un matériaux inerte par exemple des billes de stéatite, d'alumine de silice, de silice alumine, de carbure de silicium dans le cas d'un lit fixe. Dans le cas d'un lit fluidisé ou d'un lit fluidisé circulant le catalyseur peut être mis en forme par exemple par atomisation en présence d'un liant tel que la silice pour lui conférer la tenue mécanique nécessaire.

De façon préférée, on utilisera un réacteur à lit fixe contenant le catalyseur.

On peut alors avantageusement conduire l'oxydation du méthanol (ou de l'éthanol ou un autre alcool léger) dans un réacteur d'oxydation à lit fixe contenant le catalyseur pour obtenir un effluent que l'on adresse à une étape de séparation. Dans cette étape on obtient, d'une part en tête, un effluent de gaz légers comprenant le cas échéant le ou les gaz diluants, CO, CO₂, l'azote de l'air (N₂) lorsque celui-ci a été utilisé comme gaz contenant l'oxygène moléculaire, et l'O₂ résiduaire et d'autre part en pied, l'effluent de méthylal (acétal ou dialcoxyalcane) et d'eau qui est adressé à une étape de distillation pour séparer le dialcoxyalcane recherché en tête et l'eau en pied. Au moins une partie dudit effluent de gaz légers peut être utilisée dans la chaudière.

On peut utiliser de l'oxygène moléculaire ou de l'air enrichi en oxygène comme oxydant et du méthane comme diluant additionnel on obtient alors un effluent de gaz légers comprenant CH₄, CO, CO₂, N₂ et O₂ résiduaire que l'on peut le cas échéant recycler à l'entrée du réacteur d'oxydation et/ou adresser à une étape de purification pour séparer un effluent de CO et/ou CO₂ et/ou O₂ avant de le recycler à l'entrée du réacteur d'oxydation.

Comme cela a été indiqué à plusieurs reprises tous ces procédés d'oxydation d'alcools, et donc de combustibles peuvent être conduits selon les choix de compositions du mélange ternaire dans des conditions d'inflammabilité du mélange alcool-oxygène. Ces conditions ne sont pas un obstacle dirimant à une exploitation industrielle mais nécessitent des précautions opératoires qui en raison de leur coût doivent autant que possible être évitées. Il est donc préférable d'opérer dans des conditions de sécurité strictes c'est-à-dire en veillant à ne pas travailler dans la zone d'inflammabilité du mélange alcool-oxygène. Pour ce faire, on peut se référer à certaines déterminations de cette zone dans différents cas tenant compte des composants du mélange, de la température opératoire et de la pression.

Pour déterminer les conditions de réaction optimales en dehors du domaine d'inflammabilité on pourra se référer à différentes publications sur le sujet. Outre le « Catalyst Hanbook » page 498, et l'ouvrage « Catalyse de Contact» page 400, déjà mentionnés, on peut citer l'article de Michael G. Zabetakis « Flammability Characteristics of Combustible Gases and Vapors », Bureau of Mines Bulletin 627, pages 66à 68 et le Technical report ISA-TR12.13.01-1999 « Flammability Characteristics of Combustible Gases and Vapors » fig..75 et 76 et Table 13.

Dans le procédé de l'invention les zones préférées sont les Zones dans lesquelles il est possible de travailler avec de hautes teneurs à la fois en alcools (30 à 40, voire 50 ou 60 % en volume) et en oxygène, de l'ordre de 15 % tout en travaillant avec de l'air comme source d'oxygène en s'affranchissant de l'utilisation d'une source importante de gaz inerte. Il est à noter que la teneur maximum en O₂ dépend de l'alcool et elle s'élève avec le nombre de carbones de l'alcool.

On préfère utiliser un gaz oxydant riche en air afin de réduire les consommations d'électricité au niveau des compresseurs de gaz. Dans cette configuration, il n'est pas nécessaire de recycler des gaz de la réaction appauvris en oxygène pour diluer l'oxygène de l'air de réaction et donc en simplifiant le procédé.

Ce diagramme ternaire peut être transposé d'une part avec les mêmes constituants à d'autres conditions de température et de pression et d'autre part à d'autres alcools en se référant aux publications citées ci-dessus et notamment celle de Zebetakis. A la page 67 de cette publication figure un tableau d'où l'on peut déduire les teneurs maximum en oxygène selon l'alcool mis en oeuvre.

Le méthylal trouve de nombreuses applications dans divers domaines en raison de ses propriétés remarquables : un pouvoir solvant exceptionnel ; son caractère amphiphile : le méthylal est à la fois hydrophile et lipophile ; une faible viscosité ; une faible tension de surface ; et une vitesse d'évaporation particulièrement élevée.

Les domaines d'application du méthylal sont notamment les suivants : aérosols pour des applications cosmétiques et techniques ; peintures et vernis avec le méthylal comme solvant ; agents de décollage des peintures ; solvants de nettoyage et de dégraissage ; produits pharmaceutiques avec le méthylal comme support ou comme réactif ; dans la synthèse des résines; adhésifs à séchage rapide ; extraction d'arômes, produits aromatiques et parfums ; additifs aux carburants diesel ; insecticides ; cellules électrochimiques où le métliylal est une réactif dans la production de polyoxyméthylènediméthyléthers utilisés comme combustibles dans les piles à combustible.

Le diéthylacétal ou acétaldéhyde acétal, également appelé 1,1-diéthoxyéthane, est une matière première importante pour les industries du parfum et des produits pharmaceutiques. Ajouté aux parfums, il augmente leur résistance à l'oxydation et par conséquent leur durée de vie tandis qu'il joue le rôle d'exhausteur de goût dans les spiritueux. Il présente également de nombreuses applications dans l'industrie chimique et pharmaceutique où il est utilisé en tant que solvant mais aussi comme intermédiaire dans la chimie de synthèse pour la protection des groupements carbonyles des cétones et aldéhydes. De plus, il est également une molécule clé dans la synthèse de composés chimiques variés comme les alkyl vinyl éthers (utilisés comme solvants organiques de la cellulose et de ses dérivés, dans les parfums et résines synthétiques ainsi que dans les adhésifs) ou bien encore les amides d'acide N-vinylcarboxylique (matières premières des polymères hydrophiles utilisés dans les composés électroniques, les télévisions, les équipements de voitures et les imprimantes.

Le 1,1-diéthoxyéthane offre de nombreux avantages comme additif de carburant tant dans la formulation des essences que dans celle des gasoils.

Il peut être également utilisé comme additif oxygéné pour le carburant diesel car il diminue de façon drastique les émissions de particules et les NOₓ tandis qu'il maintient voire augmente l'indice de cétane et facilite ainsi la combustion des produits finaux sans diminuer les qualités d'allumage. Il faut noter qu'un haut indice de cétane indique la facilité d'un carburant à s'enflammer après avoir été injecté dans le cylindre de combustion d'un moteur diesel. Par ailleurs, le 1,1-diéthoxyéthane peut également être utilisé comme intermédiaire pour former des acétals de glycérols utilisés dans les carburants

Les exemples suivants illustrent encore la présente invention sans toutefois en limiter la portée.

### Exemple 1: Evaluation des catalyseurs

L'évaluation des catalyseurs est effectuée dans un réacteur à lit fixe. Le flux d'hélium et d'oxygène est régulé par des débitmètres massiques. Le flux de gaz passe dans un évaporateur/saturateur contenant le méthanol. L'évaporateur est soit à température ambiante soit chauffé par des rubans chauffants. La température du saturateur est ajustée pour contrôler la pression partielle de méthanol. La température du mélange gazeux est contrôlée par un thermocouple en tête du saturateur.

Le mélange gazeux est ensuite envoyé au réacteur qui est placé dans un four. La température de réaction est mesurée à l'aide d'un thermocouple qui est dans le lit catalytique.

Les effluents gazeux sont analysés par chromatographie phase gaz en ligne à l'aide d'un microGC équipé de 2 colonnes (Molecular sieve et Plot U).

Les catalyseurs sont broyés et la fraction de granulométrie 250 microns a été mélangée avec une quantité double de carbure de silicium de même granulométrie et placée dans les réacteurs en verre.

L'étalonnage du MicroGC a été effectué avec des mélanges des gaz de référence, et l'étalonnage pour les produits condensables (diméthoxyméthane, méthanol, formiate de méthyle) a été effectué en utilisant l'évaporateur saturateur.

### Exemple 2: Réaction d'oxydation du méthanol.

151 mg d'un catalyseur molybdate de fer *MFM3-MS* fourni par MAPCO, ayant un rapport atomique Mo/Fe de 2,5, ont été mélangés avec 300 mg de carbure de silicium et chargés dans le réacteur. Catalyseur *MFM3-MS :* diamètre extérieur = 3.9 mm, diamètre interne = 1.85 mm, hauteur = 4.04 mm

Le catalyseur est tout d'abord activé sous flux d'Hélium/Oxygène (48 Nml/min.- 12 Nml/min) à 340 °C pendant 15 heures et 30 minutes. Ensuite, la température est ramenée à 250 °C et l'acquisition des données commence. Après stabilisation, les performances du catalyseur sont enregistrées. Ensuite la température du catalyseur est augmentée par plateaux et à chaque niveau (260, 271 et 281 °C) des données sont prises.

Les débits d'oxygène et d'hélium sont respectivement de 6,7 et de 26,4 Nml/min et la concentration du méthanol est ajustée à 37 %. (conditions : Méthanol/O₂/inerte : 37/13/50) pour une VVH de 22 000 ml.h⁻¹.g⁻¹.

Les résultats en conversions et sélectivités obtenues lors de l'oxydation catalytique du méthanol sont reportés dans le tableau 1 (DMM = méthylal ; F = formol ; DME = diméthylether ; MF = méthylformate ; CO = monoxyde de carbone ; CO₂ = dioxyde de carbone).

**Tableau 1 :**

| | | | Sélectivités (%) | | | | | | |
|---|---|---|---|---|---|---|---|---|---|
| Catalyseur | Température (°C) | Conversion (%) | DMM | F | DME | MF | CO | CO₂ | Total |
| MFM3-MS | 250 | 25,3 | 94,3 | 0,1 | 4,9 | 0,6 | - | - | 100 |
| | 260 | 32,3 | 94,3 | 0,3 | 4,9 | 0,6 | - | - | 100 |
| "FeMo" | 271 | 46,5 | 92,7 | 1,3 | 5,2 | 0,7 | 0,1 | - | 100 |
| | 281 | 55,7 | 89,8 | 4,2 | 5,3 | 0,6 | 0,1 | - | 100 |

### Exemple 3 : Réaction d'oxydation du méthanol.

La réaction est conduite avec un catalyseur commercial : ACF-4S (de type Molybdate de Bismuth-Fer) de Nippon Shokubai. 150 mg du catalyseur commercial cité ci-dessus ont été mélangés avec 300 mg de carbure de silicium, puis chargés dans le réacteur.

Le catalyseur est tout d'abord activé sous flux d'Hélium/Oxygène (48 Nml/min - 12 Nml/min) à 340 °C pendant 15 heures et 30 minutes Ensuite, la température est ramenée à 236 °C et l'acquisition des données commence. Après stabilisation, les performances du catalyseur sont enregistrées. Ensuite la température du catalyseur est augmentée par plateaux et à chaque niveau des données sont prises.

Les débits d'oxygène et d'hélium sont respectivement de 6,7 et de 26,4 Nml/min et la concentration du méthanol est ajustée à 37 %. (conditions : Méthanol/O₂/inerte : 37/13/50) pour une VVH de 22 000 ml.h⁻¹.g⁻¹.
avec DMM = methylal ; F = formol ; DME = diméthylether ; MF = méthylformate ; CO = monoxyde de carbone ; CO₂ = dioxyde de carbone.

Les résultats obtenus en conversion et sélectivités sont reportés dans le tableau 2 suivant:

**Tableau 2**

| | | | Sélectivités (%) | | | | | | |
|---|---|---|---|---|---|---|---|---|---|
| Catalyseur | Température (°C) | Conversion (%) | DMM | F | DME | MF | CO | CO₂ | Total |
| ACF-4S de | 250 | 6,7 | 88,1 | 5,6 | 5,8 | 0,5 | - | - | 100 |
| Nippon | 259 | 9,1 | 87,9 | 5,8 | 5,6 | 0,6 | - | - | 100 |
| Shokubai | 271 | 13,0 | 88,5 | 5,5 | 5,3 | 0,6 | - | 0,0 | 100 |
| "BiMo" | 280 | 16,7 | 88,3 | 5,9 | 5,1 | 0,7 | - | 0,0 | 100 |

### Exemple 4 comparatif:

On réalise l'oxydation du méthanol, conformément aux procédés de l'art antérieur avec 150 mg du catalyseur commercial Molybdate de Fer *MFM3-MS* (MAPCO) qui ont été mélangés avec 300 mg de carbure de silicium, puis chargés dans le réacteur.

Le catalyseur est tout d'abord activé sous flux d'Hélium/Oxygène (48 Nml/min - 12 Nml/min) à 340 °C pendant 15 heures et 30 minutes. Ensuite, la température est ramenée à 236 °C et l'acquisition des données commence. Après stabilisation, les performances du catalyseur sont enregistrées. Ensuite la température du catalyseur est augmentée par plateaux et à chaque niveau (255 et 265) des données sont prises.

Les débits d'oxygène et d'hélium sont respectivement de 4,7 et de 47,6 Nml/min et la concentration du méthanol est ajustée à 5 % du milieu réactionnel. (Méthanol/O₂/inerte : 5/8.5/86.5).

Les résultats sont reportés dans le tableau 3 suivant.

**Tableau 3**

| Température (°C) | CH₃OH conversion (%) | DMM sélectivité (%) | DMM Rendement (%) |
|---|---|---|---|
| 236 | 41 | 36 | 15 |
| 255 | 57 | 20 | 11 |
| 265 | 67 | 11 | 7 |

Comme on peut le voir par comparaison entre les tableaux 1 et 3, les résultats obtenus en utilisant une faible pression partielle de méthanol conduisent à des sélectivités et des rendements en diméthoxyméthane beaucoup plus faibles que lorsque l'on utilise des pressions partielles élevées. Ces résultats sont d'autant plus inattendus que les conversions peuvent être maintenues à un niveau élevé.

### Exemple 5 : Conditions opératoires de l'oxydation sélective de l'éthanol

Le catalyseur est testé dans un réacteur à lit fixe. Les débits des gaz hélium et oxygène sont régulés par un régulateur de débit massique. Le mélange gazeux passe à travers un évaporateur / saturateur rempli avec de l'éthanol. L'évaporateur peut-être à température ambiante ou chauffé par un cordon chauffant. La température du saturateur est ajustée et contrôlée afin d'obtenir la pression partielle en éthanol souhaitée. La température est mesurée à l'aide d'un thermocouple en sortie du saturateur.

Le mélange réactionnel alimente le réacteur qui est placé dans un four. La température de la réaction est mesurée par un thermocouple placé dans le lit catalytique.

Les effluents gazeux sont analysés en ligne par une micro-GC équipée de trois colonnes (Tamis moléculaire, Plot U et OV-1).

Un flux d'hélium et d'oxygène passe à travers l'évaporateur / saturateur ajustés aux températures adéquates permettant d'obtenir la composition désirée en éthanol/oxygène / hélium. Le catalyseur est mélangé avec la quantité quadruple de carbure de silicium dans le réacteur en verre.

L'étalonnage de la micro-GC est effectué avec des mélanges gazeux de référence et les produits condensables sont étalonnés en utilisant l'évaporateur / saturateur.

151 mg du catalyseur MFM3-MS (fourni par MAPCO) sont mélangés avec 600 mg de carbure de silicium et sont chargés dans le réacteur.

Le catalyseur est activé à une température de 340 °C sous un mélange hélium/oxygène (48 Nml.min⁻¹ / 12 Nm1.min⁻¹) pendant 12 heures. Ensuite la température est descendue à 200°C et les données sont enregistrées. Après stabilisation, l'efficacité du catalyseur est testée. Après acquisition des données la température du catalyseur est augmentée jusqu'à la température suivante, 228°C puis 260°C où les données sont enregistrées.

Les débits d'oxygène et d'hélium sont respectivement de 12,7 et 51 Nml.min⁻¹ et la température du saturateur est ajustée pour obtenir une fraction molaire en éthanol de 2 % (Ethanol/O₂/inerte = 2/19.5/78.5).

Les résultats en matière de conversions et sélectivités obtenues: lors de l'oxydation catalytique de l'éthanol exprimés comme suit: A = acétaldéhyde; DEE = 1.1 diéthoxyéthane ; EE = éther éthylique ; AE = acétate d'éthyle ; AA = acide ascétique E = éthylène ; CO = monoxyde de carbone ; CO₂ = dioxyde de carbone sont donnés dans le tableau 4.

**Tableau 4 :**

| | | Sélectivités en carbone (%) | | | | | | | |
|---|---|---|---|---|---|---|---|---|---|
| Température (°C) | Conversion d'éthanol (%) | A | DEE | EE | AE | AA | E | CO | CO₂ |
| 200 | 31.2 | 92.5 | 6.5 | 1 | - | - | - | - | - |
| 228 | 61.6 | 98.5 | - | 1.5 | - | - | - | - | - |
| 260 | 91.5 | 93.2 | - | 0.9 | 1 | - | 1.9 | 1.4 | 1.6 |

Dans ces conditions de fonctionnement le catalyseur est très sélectif pour donner de l'acétaldéhyde.

### Exemple 7 :

150 mg du catalyseur *MFM3-MS* (MAPCO) sont mélangés avec 600 mg de carbure de silicium et sont chargés dans le réacteur.
Le catalyseur est activé à une température de 340 °C sous un mélange hélium/oxygène (48 Nml.min⁻¹ / 12 Nml.min⁻¹) pendant 12 heures. Ensuite la température est descendue à 200°C et les données sont enregistrées. Après stabilisation, l'efficacité du catalyseur est testée. Après acquisition des données, la température du catalyseur est augmentée jusqu'à la température suivante, 228°C puis 260°C où les données sont enregistrées.

Les débits d'oxygène et d'hélium sont respectivement de 0,3 et 63,4 Nml.min⁻¹ et la température du saturateur est ajustée pour obtenir une fraction molaire en éthanol de 2 % pour obtenir EOH/O₂/inerte= 2/0.5/97.5.

Les résultats en conversions et sélectivités obtenues lors de l'oxydation catalytique de l'éthanol sont donnés dans le tableau 5.

**Tableau 5**

| | | Sélectivités en carbone (%) | | | | | | | |
|---|---|---|---|---|---|---|---|---|---|
| Température (°C) | Conversion d'éthanol (%) | A | DEE | EE | AE | AA | E | CO | CO₂ |
| 200 | 18.4 | 100 | - | - | - | - | - | - | - |
| 228 | 41.4 | 97.5 | - | 2.5 | - | - | - | - | - |
| 260 | 63.5 | 95.7 | - | 2.1 | - | - | 2.2 | - | - |

Bien que le catalyseur soit alimenté avec un flux moins riche en oxygène que dans le cas de l'exemple précédent, il reste très sélectif pour la production d'acétaldéhyde.

### Exemple 8 :

150 mg du catalyseur *MFM3-MS* (MAPCO) sont mélangés avec 600 mg de carbure de silicium et sont chargés dans le réacteur.

Le catalyseur est activé à une température de 340 °C sous un mélange hélium/oxygène (48 Nml.min⁻¹ / 12 Nm1.min⁻¹) pendant 12 heures. Ensuite la température est descendue à 201°C et les données sont enregistrées. Après stabilisation, l'efficacité du catalyseur est testée. Après acquisition des données, la température du catalyseur est augmentée jusqu'à la température suivante, 231 °C puis 260°C où les données sont enregistrées.

Les débits d'oxygène et d'hélium sont respectivement de 4,6 et 41 Nml.min⁻¹ et la température du saturateur est ajustée pour obtenir une fraction molaire en éthanol de 30 %, Ethanol/O₂/He = 30/7/63.

Les résultats en conversions et sélectivités obtenues lors de l'oxydation catalytique de l'éthanol sont donnés dans le tableau 6.

**Tableau 6**

| | | Sélectivités en carbone (%) | | | | | | | |
|---|---|---|---|---|---|---|---|---|---|
| Température (°C) | Conversion d'éthanol (%) | A | DEE | EE | AE | AA | E | CO | CO₂ |
| 201 | 5 | 62 | 36 | 2 | - | - | - | - | - |
| 231 | 10.8 | 68.5 | 28.5 | 3 | - | - | - | - | - |
| 260 | 25.6 | 77.6 | 17.7 | 4.1 | - | - | 0.6 | - | - |

Dans ces conditions de fonctionnement le catalyseur produit du diéthoxyéthane qui n'est pas détecté dans les conditions de faibles pressions partielles en éthanol.

### Exemplé 9 :

75 mg du catalyseur *MFM3-MS* (MAPCO) sont mélangés avec 300 mg de carbure de silicium et sont chargés dans le réacteur.

Le catalyseur est activé à une température de 340 °C sous un mélange hélium/oxygène (48 Nml.min⁻¹ / 12 Nm1.min⁻¹) pendant 12 heures. Ensuite la température est descendue à 199°C et les données sont enregistrées. Après stabilisation, l'efficacité du catalyseur est testée. Après acquisition des données, la température du catalyseur est augmentée jusqu'à la température suivante, 230°C puis 260°C où les données sont enregistrées.

Les débits d'oxygène et d'hélium sont respectivement de 4,6 et 41 Nml.min⁻¹ et la température du saturateur est ajustée pour obtenir une fraction molaire en méthanol de 30 %, Ethanol/O₂/He = 30/7/63.

Les résultats en conversions et sélectivités obtenues lors de l'oxydation catalytique de l'éthanol sont donnés dans le tableau 7.

**Tableau 7**

| | | Sélectivités en carbone (%) | | | | | | | |
|---|---|---|---|---|---|---|---|---|---|
| Température (°C) | Conversion d'éthanol (%) | A | DEE | EE | AE | AA | E | CO | CO₂ |
| 199 | 2.4 | 26 | 72.7 | 1.3 | - | - | - | - | - |
| 230 | 8.4 | 36.3 | 61.7 | 2 | - | - | - | - | - |
| 260 | 17.2 | 55.9 | 40.4 | 3.3 | - | - | 0.4 | - | - |

Dans des conditions de forte VVH (faible temps de contact), double de celle de l'exemple 8, le catalyseur s'avère être sélectif en diéthoxyéthane.

### Exemple 10 :

150 mg d'un catalyseur *MFM3-HS* (MAPCO) sont mélangés avec 600 mg de carbure de silicium et sont chargés dans le réacteur.

*MFM3-HS* fourni par MAPCO se distingué du précédent *MFM3-MS* notamment par ses dimensions mais aussi par son activité : diamètre extérieur =4.35 mm, diamètre interne = 1.85 mm, hauteur = 4.44 mm.

Le catalyseur est activé à une température de 340 °C sous un mélange hélium/oxygène (48 Nml.min⁻¹ / 12 Nml.min⁻¹) pendant 12 heures. Ensuite la température est descendue à 198°C et les données sont enregistrées. Après stabilisation, l'efficacité du catalyseur est testé. Après acquisition des données, la température du catalyseur est augmentée jusqu'à la température suivante, 230°C puis 260°C où les données sont enregistrées.

Les débits d'oxygène et d'hélium sont respectivement de 4,6 et 41 Nml.min⁻¹ et la température du saturateur est ajustée pour obtenir une fraction molaire en éthanol de 30% Ethanol/O₂/He = 30/7/63. Les résultats en conversions et sélectivités obtenues lors de l'oxydation catalytique de l'éthanol sont donnés dans le tableau 8.

**Tableau 8**

| | | Sélectivités en carbone (%) | | | | | | | |
|---|---|---|---|---|---|---|---|---|---|
| Température (°C) | Conversion d'éthanol (%) | A | DEE | EE | AE | AA | E | CO | CO₂ |
| 198 | 2.4 | 23.8 | 74.7 | 1.5 | - | - | - | - | - |
| 230 | 7 | 37.2 | 60.4 | 2.2 | - | - | 0.2 | - | - |
| 260 | 17.1 | 57.9 | 37.9 | 3.7 | - | - | 0.4 | - | 0.1 |

Là encore le catalyseur s'avère sélectif en diéthoxyéthane.

## Revendications

1. Procédé de fabrication d'un produit d'oxydation partielle d'un alcool léger, sous forme d'un dialcoxyalcane dans lequel un alcool léger comprenant de 1 à 4 atomes de carbone est soumis à une oxydation par contact en phase gazeuse avec de l'oxygène ou un gaz contenant de l'oxygène moléculaire en présence d'un catalyseur répondant à la composition suivante :
**Mo₁₂FeₐX_{y} X¹_{b}:X²_{c}X³_{d} X⁴ₑ X_{f}⁵ Oₓ**
dans laquelle Mo = molybdène ; O = oxygène ; Fe = fer ; X¹ = au moins un élément choisi parmi le chrome, le nickel, le cobalt, le manganèse, l'étain et le cuivre, X² = au moins un élément choisi parmi le bismuth, l'antimoine, le tellure, l'indium, l'aluminium et le silicium et , X³ = au moins un élément choisi parmi le phosphore, le tungstène, le titane, le vanadium, le tantale et le niobium; X⁴ = au moins un élément choisi parmi les métaux alcalino-terreux, le lanthane ou le cérium ; X⁵ est au moins un élément choisi parmi les métaux alcalins ; et a, b, c d, e sont des indices dont les valeurs sont 1,5≤a≤8 ; 0≤b≤4 ; 0≤c≤5 ; 0≤d≤2 ; 0≤e≤2 ; 0<f<2 et x est une valeur numérique déterminée par le degré d'oxydation des autres éléments, et **caractérisé par le fait que**, au sein du milieu réactionnel la pression partielle en alcool est comprise entre 15 et 80 % et de préférence comprise entre 20 et 50 % et celle de l'oxygène comprise entre 2 et 20 %, le ratio des pressions partielles O₂/Alcool étant inférieur ou égal à 1 et de préférence compris entre 0,5/6 et 1, le reste du milieu étant constitué d'un gaz inerte vis à vis de la réaction.

2. Procédé selon la revendication 1, **caractérisé par le fait que** l'alcool léger est choisi parmi le méthanol, l'éthanol, le propanol, l'isopropanol, le n-butanol et le 2-butanol.

3. Procédé selon l'une des revendications 1 à 2 **caractérisé par le fait que** le rapport molaire de l'oxygène, calculé en tant qu'O₂ à l'alcool léger est comprise entre 0,5/6 à 1/1, de préférence entre 1,2/6 et 0,9/1.

4. Procédé selon l'une des revendications 1 à 3 **caractérisé en ce que** la réaction sera généralement conduite à une température comprise entre 10 et 400°C et sous une pression comprise entre 50 et 1 000 kPa et avec une vitesse spatiale d'introduction du mélange réactionnel notamment comprise entre 2 000 et 100 000 h⁻¹.

5. Procédé selon l'une des revendications 1 à 4 **caractérisé par le fait que** l'alcool léger est le méthanol ou l'éthanol et le produit d'oxydation partielle est le méthylal ou l'acétal et que l'on conduit l'oxydation par contact en phase vapeur à une température de 10 à 400°C, de préférence de 100 à 350°C, et de façon davantage préférée de 200 à 300°C et à une pression de 50 à 1 000 kPa et de préférence de 100-500 kPa.

6. Procédé selon la revendication 5 **caractérisé par le fait que** la concentration de l'alcool léger dans le courant gazeux est compris entre 25 et 40 et de préférence compris entre 30 et 37 % et celle de l'oxygène est telle que le ratio O₂/alcool est supérieur à 1/6 et de préférence compris entre 1,2 /6 et 0,9 /1.

7. Procédé selon l'une des revendications 1 à 6 **caractérisé en ce que** X₂ est le bismuth.

8. Procédé selon l'une des revendications 1 à 7 **caractérisé par le fait que** l'on utilise un catalyseur choisi parmi les oxydes mixtes de formules :
Mo₁₂BiFe_{3.7}Co_{4.7}Ni_{2.6}K_{0.09}Sb₁Si_{7.9}Oₓ, Mo₁₂BiFe_{3.7}Co_{4.7}Ni_{2.6}K_{0.09}Ti_{0.5}Si₁₉Oₓ et MoO₃-Fe₂(MoO₄)₃.

9. Procédé selon l'une des revendications 5 à 8, **caractérisé par le fait que** la vitesse spatiale d'introduction du mélange gazeux est de 2 000-100 000 h⁻¹, de préférence de 11 000-44 000 h⁻¹.

10. Procédé selon l'une des revendications 1 à 9 **caractérisé par le fait que** l'on conduit l'oxydation de l'alcool léger dans un réacteur d'oxydation à lit fixe contenant le catalyseur.

11. Procédé selon la revendication 10 **caractérisé par le fait qu'**à la sortie du réacteur on obtient un effluent que l'on adresse à une étape de séparation afin obtenir, d'une part, en tête un effluent de gaz légers comprenant le ou les gaz diluants, CO, CO₂, l'azote de l'air (N₂) lorsque celui-ci a été utilisé comme gaz contenant l'oxygène moléculaire, l'O₂ résiduaire, et en pied un effluent de dialcoxyalcane et d'eau adressé à une étape de distillation pour obtenir séparer le dialcoxyalcane et l'eau, au moins une partie dudit effluent de gaz légers pouvant le cas échéant être utilisée en chaudière.

## Patentansprüche

1. Verfahren zur Herstellung eines Partialoxidationsprodukts eines leichten Alkohols in Form eines Dialkoxyalkans, wobei ein leichter Alkohol mit 1 bis 4 Kohlenstoffatomen einer Oxidation unterworfen wird, indem er in der Gasphase in Gegenwart eines Katalysators der folgenden Zusammensetzung:
**Mo₁₂ Feₐ X¹_{b} X²_{c} X³_{d} X⁴ₑ X⁵_{f} Oₓ**
worin Mo = Molybdän; O = Sauerstoff; Fe = Eisen; X¹ = mindestens ein aus Chrom, Nickel, Cobalt, Mangan, Zinn und Kupfer ausgewähltes Element; X² = mindestens ein aus Bismut, Antimon, Tellur, Indium, Aluminium und Silicium ausgewähltes Element, und X³ = mindestens ein aus Phosphor, Wolfram, Titan, Vanadium, Tantal und Niob ausgewähltes Element; X⁴ = mindestens ein aus Erdalkalimetallen, Lanthan oder Cer ausgewähltes Element; X⁵ für mindestens ein aus Alkalimetallen ausgewähltes Element steht; und a, b, c, d und e für Indices stehen, dessen Werte 1,5 ≤ a ≤ 8; 0 ≤ b ≤ 4; 0 ≤ c ≤ 5; 0 ≤ d ≤ 2; 0 ≤ e ≤ 2; 0 ≤ f ≤ 2 betragen, und x für einen durch die Oxidationsstufe der anderen Elemente bestimmten Zahlenwert steht, mit Sauerstoff oder einem molekularen Sauerstoff enthaltenden Gas in Kontakt gebracht wird, und **dadurch gekennzeichnet, dass** im Reaktionsmedium der Alkohol-Partialdruck zwischen 15 und 80% und vorzugsweise zwischen 20 und 50% liegt und der Sauerstoff-Partialdruck zwischen 2 und 20% liegt, wobei das Verhältnis der O₂/Alkohol-Partialdrücke kleiner gleich 1 ist und vorzugsweise zwischen 0,5/6 und 1 liegt, wobei der Rest des Mediums aus einem gegenüber der Reaktion inerten Gas bestehen kann.

2. Verfahren nach Anspruch 1, **dadurch gekennzeichnet, dass** der leichte Alkohol aus Methanol, Ethanol, Propanol, Isopropanol, n-Butanol und 2-Butanol ausgewählt wird.

3. Verfahren nach einem der Ansprüche 1 bis 2, **dadurch gekennzeichnet, dass** das Molverhältnis von Sauerstoff, berechnet als O₂, zu leichtem Alkohol zwischen 0,5/6 und 1/1, vorzugsweise zwischen 1,2/6 und 0,9/1, liegt.

4. Verfahren nach einem der Ansprüche 1 bis 3, **dadurch gekennzeichnet, dass** die Reaktion im Allgemeinen bei einer Temperatur zwischen 10 und 400°C und unter einem Druck zwischen 50 und 1000 kPa und mit einer Raumgeschwindigkeit der Eintragung der Reaktionsmischung, die insbesondere zwischen 2000 und 100.000 h⁻¹ liegt, durchgeführt wird.

5. Verfahren nach einem der Ansprüche 1 bis 4, **dadurch gekennzeichnet, dass** es sich bei dem leichten Alkohol um Methanol oder Ethanol handelt und es sich bei dem Partialoxidationsprodukt um Methylal oder Acetal handelt und die Oxidation durch Kontakt in der Dampfphase bei einer Temperatur von 10 bis 400°C, vorzugsweise 100 bis 350°C und vorteilhafterweise 200 bis 300°C und bei einem Druck von 50 bis 1000 kPa und vorzugsweise 100-500 kPa durchgeführt wird.

6. Verfahren nach Anspruch 5, **dadurch gekennzeichnet, dass** die Konzentration des leichten Alkohols in dem Gasstrom zwischen 25 und 40% und vorzugsweise zwischen 30 und 37% liegt und die Konzentration des Sauerstoffs so beschaffen ist, dass das O₂/Alkohol-Verhältnis größer als 1/6 ist und vorzugsweise zwischen 1,2/6 und 0,9/1 liegt.

7. Verfahren nach einem der Ansprüche 1 bis 6, **dadurch gekennzeichnet, dass** X₂ für Bismut steht.

8. Verfahren nach einem der Ansprüche 1 bis 7, **dadurch gekennzeichnet, dass** ein aus den Mischoxiden der Formeln Mo₁₂BiFe₃, ₇Co₄, ₇Ni₂, ₆K₀, ₀₉Sb₁Si₇, ₉Oₓ, Mo₁₂BiFe₃, ₇Co₄, ₇Ni₂, ₆K₀, ₀₉Ti₀, ₅Si₁₉Oₓ und MoO₃-Fe₂ (MoO₄)₃ ausgewählter Katalysator verwendet wird.

9. Verfahren nach einem der Ansprüche 5 bis 8, **dadurch gekennzeichnet, dass** die Raumgeschwindigkeit der Eintragung der gasförmigen Reaktionsmischung 2000-100.000 h⁻¹ und vorzugsweise 11.000-44.000 h⁻¹ beträgt.

10. Verfahren nach einem der Ansprüche 1 bis 9, **dadurch gekennzeichnet, dass** die Oxidation des leichten Alkohols in einem Festbett-Oxidationsreaktor, der den Katalysator enthält, durchgeführt wird.

11. Verfahren nach Anspruch 10, **dadurch gekennzeichnet, dass** am Ausgang des Reaktors ein Austragsstrom erhalten wird, der einem Trennschritt unterworfen wird, bei dem zum einen am Kopf ein Austragsstrom leichter Gase, umfassend das verdünnende Gas bzw. die verdünnenden Gase, CO, CO₂, Stickstoff aus der Luft (N₂), wenn diese als molekularen Sauerstoff enthaltendes Gas verwendet worden ist, restliches O₂, und zum anderen am Sumpf einen Austragsstrom von Dialkoxyalkan und Wasser erhalten wird, welcher einem Destillationsschritt zugeführt wird, bei dem das Dialkoxyalkan und das Wasser getrennt werden, wobei mindestens ein Teil des Austragsstrom leichter Gase gegebenenfalls in einem Dampfkessel verwendet werden kann.

## Claims

1. Method for producing a partial oxidation product of a light alcohol, in the form of a dialkoxyalkane, in which a light alcohol comprising from 1 to 4 carbon atoms is subjected to oxidation by contact in the gas phase with oxygen or a gas containing molecular oxygen in the presence of a catalyst corresponding to the following composition:
**Mo₁₂ Feₐ X¹_{b} X²_{c} X³_{d} X⁴ₑ X⁵_{f} Oₓ**
in which Mo = molybdenum; 0 = oxygen; Fe = iron; X¹ = at least one element chosen from chromium, nickel, cobalt, manganese, tin and copper; X² = at least one element chosen from bismuth, antimony, tellurium, indium, aluminium and silicon, X³ = at least one element chosen from phosphorus, tungsten, titanium, vanadium, tantalum and niobium; X⁴ = at least one element chosen from alkaline-earth metals, lanthanum and cerium; X⁵ is at least one element chosen from alkali metals; and a, b, c, d and e are indices whose values are 1.5 ≤ a ≤ 8; 0 ≤ b ≤ 4; 0 ≤ c ≤ 5; 0 ≤ d ≤ 2; 0 ≤ e ≤ 2; 0 ≤ f ≤ 2 and x is a numerical value determined by the degree of oxidation of the other elements, and **characterized in that**, within the reaction medium, the partial pressure of alcohol is between 15 and 80% and preferably between 20 and 50% and that of oxygen is between 2 and 20%, the ratio of the O₂/alcohol partial pressures being less than or equal to 1 and preferably between 0.5/6 and 1, the remainder of the medium being composed of a gas that is inert towards the reaction.

2. Method according to Claim 1, **characterized in that** the light alcohol is chosen from methanol, ethanol, propanol, isopropanol, n-butanol and 2-butanol.

3. Method according to either of Claims 1 and 2, **characterized in that** the molar ratio of oxygen, calculated as O₂, to the light alcohol is between 0.5/6 and 1/1, preferably between 1.2/6 and 0.9/1.

4. Method according to one of Claims 1 to 3, **characterized in that** the reaction will generally be carried out at a temperature between 10 and 400°C and under a pressure between 50 and 1000 kPa and with a space velocity for introducing the reaction mixture between, in particular, 2000 and 100 000 h⁻¹.

5. Method according to one of Claims 1 to 4, **characterized in that** the light alcohol is methanol or ethanol and the partial oxidation product is methylal or acetal and that the oxidation is carried out by contact in the vapour phase at a temperature of 10 to 400°C, preferably from 100 to 350°C, and more preferably from 200 to 300°C and at a pressure of 50 to 1000 kPa and preferably from 100 to 500 kPa.

6. Method according to Claim 5, **characterized in that** the concentration of the light alcohol in the gas stream is between 25 and 40% and preferably between 30 and 37%, and that of the oxygen is such that the O₂/alcohol ratio is greater than 1/6 and preferably between 1.2/6 and 0.9/1.

7. Method according to one of Claims 1 to 6, **characterized in that** X₂ is bismuth.

8. Method according to one of Claims 1 to 7, **characterized in that** a catalyst is used that is chosen from the mixed oxides of formulae:
Mo₁₂BiFe_{3.7}Co₄.7Ni_{2.6}K_{0.09}Sb₁Si_{7.9}Oₓ, Mo₁₂BiFe_{3.7}Co₄.7Ni_{2.6}K_{0.09} Ti_{0.5}Sᵢ₁₉Oₓ or MoO₃-Fe₂(MoO₄)₃.

9. Method according to one of Claims 5 to 8, **characterized in that** the space velocity for introducing the gaseous mixture is from 2000 - 100 000 h⁻¹, preferably from 11 000 - 44 000 h⁻¹.

10. Method according to one of Claims 1 to 9, **characterized in that** the oxidation of the light alcohol is carried out in a fixed-bed oxidation reactor containing the catalyst.

11. Method according to Claim 10, **characterized in that** an effluent is obtained at the outlet of the reactor, said effluent being subjected to a separation step in order to obtain, on the one hand, at the top, an effluent of light gases comprising the diluent gas or gases, CO, CO₂, nitrogen from the air (N₂) when air has been used as the gas containing molecular oxygen, residual O₂, and, at the bottom, an effluent of dialkoxyalkane and water subjected to a distillation step in order to separate the dialkoxyalkane and the water, at least one part of said effluent of light gases possibly, where appropriate, being used in a boiler.
